# EUROPEAN PATENT APPLICATION

(11) **EP 3 925 634 A1**
(43) Date of publication of application: **22.12.2021**
(21) Application number: 20208308.5
(22) Date of filing: 18.11.2020
(51) Int. Cl.: A61L 9/20, A61L 9/22

(54) **PHOTO-ELECTRONIC AIR DISINFECTOR**

(30) Priority: 17.06.2020 HK 32020009507; 27.07.2020 HK 22020012668
(71) Applicant: John Technology Holdings Limited, Kwun Tong, Kowloon (HK)
(72) Inventor: YUEN, Se Kit, Kowloon (HK)
(74) Representative: Murgitroyd & Company

(57) **Abstract**

The present invention provides a photo-electronic air disinfector, which comprises a housing containing a fan, a UVC light source, a photocatalyst module consisting of a structure with entire surface area coated with a photocatalyst positioned by the side of the UVC light source, an air guide unit installed with reflecting mirrors on the internal walls of the unit. When air is drawn in the air guide unit of the device, the photocatalyst after absorbing UVC light emitted by the UVC light source or reflected through the mirrors generates superoxide (O2-) and hydroxyl radical(· OH) with strong oxidation power, which can decompose organic matter and various germs in the drawn in air and render them harmless. Sterilization effect of the present invention is four-folded, firstly the air filter removing dusts and germs from the air before entering the device, secondly the photocatalysis produced by the photocatalyst module exposed to direct and reflected UVC light resulting in oxidation process for decomposing organic matter and various germs and rendering them harmless, thirdly UVC light rays and their multiple reflection through the mirrors for bacteria termination, and fourthly negative ions produced and added to the air to condense fine particles into larger ones for settling on the ground and thus reducing harmful pollutants in the air.

## Description

### FIELD OF THE INVENTION

This invention relates to a device named the photo-electronic air disinfector employing among others the process of Photocatalysis for air disinfection.

### BACKGROUND OF THE INVENTION

Photocatalysis is the process when photocatalyst like titanium dioxide coming into contact with light including UV light, an oxidation-reduction reaction occurs such that volatile organic compounds (VOC) and various germs (like mould, E.coli, Staphylococcus aureus and others) are broken down and made harmless. Photocatalysis is an effective process for use in air and water disinfection, though feasibility of its use has been limited because as a common practice coating of a photocatalyst on the wall or furniture is necessary for it to function for human benefit.

### SUMMARY OF THE INVENTION

The present invention provides a photo-electronic air disinfector, which solves the limitation that the photocatalyst must be coated on the wall or furniture for it to take effect, and enables disinfection by photocatalysis to be used in a wider environment.

The device comprises a housing containing a fan which could be a turbine extractor, an electric motor for driving the fan, a UVC light source, a photocatalyst module consisting of a structure with entire surface area coated with a photocatalyst positioned by the side of the UVC light source, an air guide unit installed with reflecting mirrors on the internal walls of the unit, a rechargeable battery, a negative-ion generator with a cathodic high-voltage carbonized fiber, an air filter which could be an activated carbon and HEPA filter, wherein:
- the housing has an air inlet grille designed for installation of an air filter for filtering the air before entering the device, and an air outlet grille for extraction of air out of the device, while in between these inlet grille and outlet grille is the air guide unit;
- the fan has the functions of drawing in air into the device through the air inlet grille, and forcing the air to go through the air guide unit, and then extracting air out of the device through the air outlet grille;
- inside the air guide unit of the device, a structure called the photocatalyst module comprising a structure with entire surface area coated with a photocatalyst is positioned by the side a UVC light source which is also installed inside the air guide unit;
- after absorbing UVC light emitted by the UVC light source, the photocatalyst generates superoxide (O2-) and hydroxyl radical(· OH) with strong oxidation power, which decomposes organic matter and various germs in the drawn in air and renders them harmless;
- air passing through the photocatalyst module will also be exposed to the UVC light emitted by a UVC light source for the extermination of bacteria and viruses;
- the UVC light emitted by a UVC light source will be constantly and repeatedly reflected by the multiple mirrors installed on the internal wall of the air guide unit, for the purposing of increasing the efficacy of the UVC sterilization such that the bacteria or viruses in air is repeatedly irradiated by UVC through multiple reflections by the mirrors.

In a preferred embodiment, the air filter of the device firstly filters out airborne impurities like dust, airborne bacteria and germs. The photocatalyst module of the device, after absorbing UVC light emitted by the UVC light source light installed inside the air guide unit, will generate superoxide (O2-) and hydroxyl radical(· OH) with strong oxidation power, which can effectively decompose organic matter and various germs and render them harmless. For example, photocatalyst can degrade indoor harmful volatile organic compounds like formaldehyde into non-toxic and harmless small molecule water and carbon dioxide. Those bacteria and germs capable of passing through the air filter and not having been deactivated by the photocatalyst module will be eliminated by UVC light emitted by the UVC light source. As the multiple mirrors surrounding the UVC light source are installed on the internal wall of the air guide unit, surviving bacteria and germs will be repeatedly irradiated by UVC light through multiple reflections by the mirrors. Negative ions are generated by a negative ion generator with a high-voltage carbonized fiber inside the device near the air outlet grille and extracted out of the device by the fan. The negative ions when coming into contact with fine particles in the air will condense them into larger particles for settling on the ground, thus reducing harmful pollutants in the air. Sterilization effect of the device is four-folded, first the air filter, second the photocatalysis by the photocatalyst module, third UVC light rays and their multiple reflection through the mirrors, and fourth negative ions added to the air.

Usual air purifier will preferably have to be placed in the middle of a room for the benefit of providing fresh air the human being inside the room. This device however is operable by a rechargeable battery, and could be carried around by the user through hanging on the neck of a person by a strap hooked to the device, enabling the user to breathe in purified air coming out of the device within a short distance.

### DESCRIPTION OF THE DRAWINGS

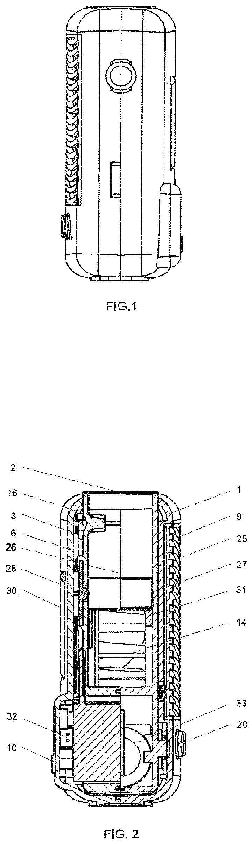

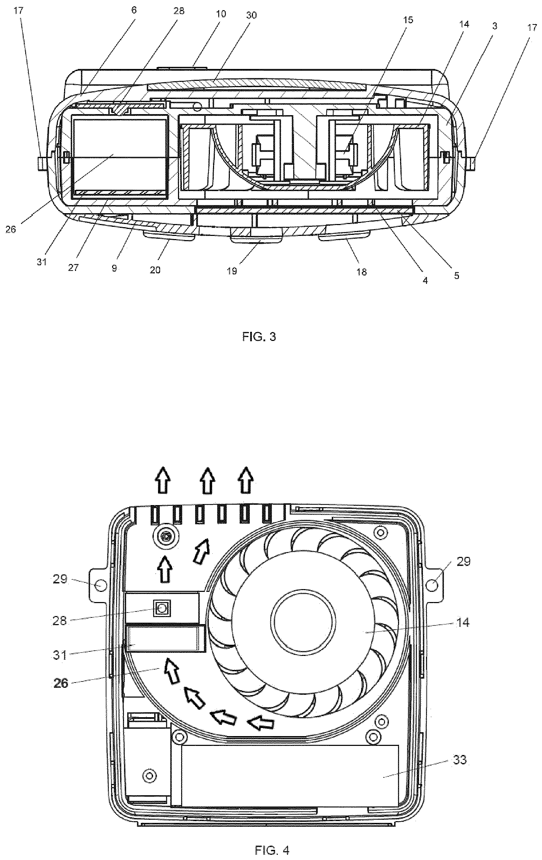

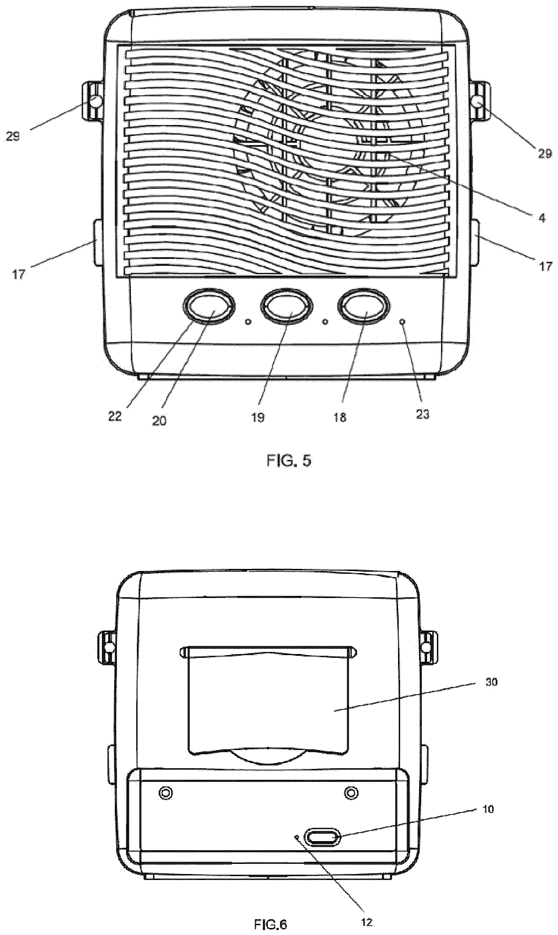

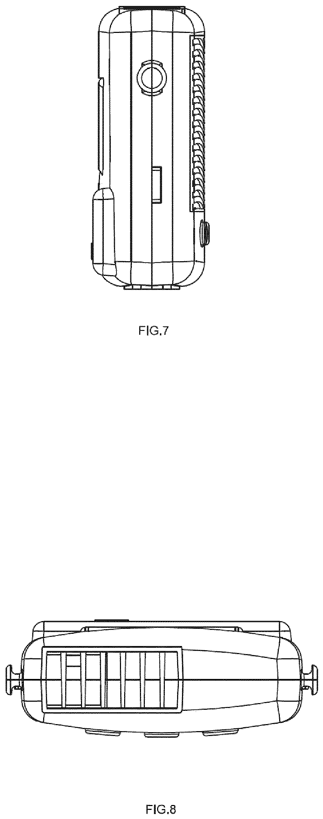

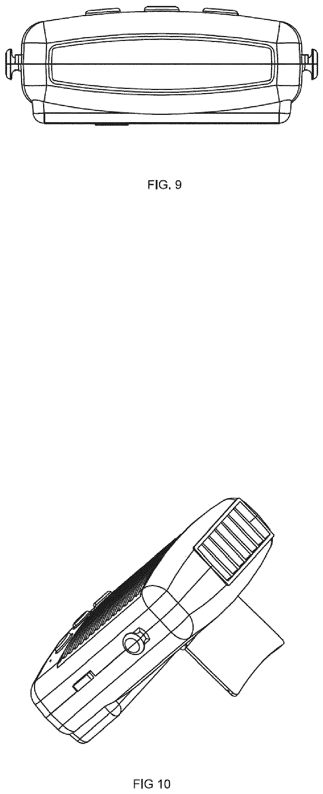

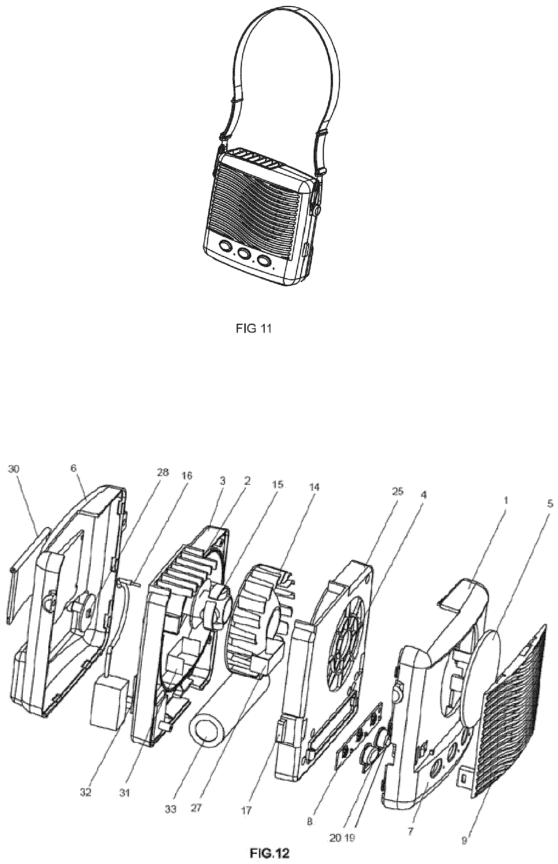

A preferred non-limiting embodiment of the present invention will now be described with reference to the accompanying diagrammatic drawings, in which: FIG 1 is a side elevation view of a photo-electronic air disinfector in accordance with a preferred embodiment of the present invention; FIG 2 is a side vertical cross-sectional view of the device depicted in FIG 1; FIG 3 is a horizontal cross-sectional view of the device depicted in FIG 1; FIG 4 is a front vertical cross-sectional view of the device depicted in FIG 1; FIG 5 is a front view of the device depicted in FIG 1; FIG 6 is a rear view of the device depicted in FIG 1; FIG 7 is a side elevation view of the other side of the device depicted in FIG 1; FIG 8 is a top view of the device depicted in FIG 1; FIG 9 is a bottom view of the device depicted in FIG 1; FIG 10 is to show the retractable stand of the device depicted in FIG 1; FIG 11 is to show the adjustable neck strap of the device depicted in FIG 1;and FIG 12 is an exploded perspective view of the device depicted in FIG 1.

### DESCRIPTION OF AN EMBODIMENT

The photo-electronic air disinfector consists of housing 1, with an air outlet grille 2 located on top of the rear inner housing 3. On the front of the inner housing 25 there is an air inlet grille 4. After ionization, air is expelled via the air outlet grille 2.

The detailed structure of the photo-electronic air disinfector is shown in FIG 2. There is an air inlet grille 4 on the top of the front of the inner housing 25. This air inlet grille 4 provides an air intake. There is an air guide unit 26 connected to the front end of the air inlet grille 4. On the bottom end of the housing 1 there is a control panel cover 7, which secures the electrical function circuit equipment 8. There is a protective cover 9 on top of the front of the housing 1, which secures the replaceable air filter 5. Close to the internal surface of the air inlet grille 4 is the fan 14 and the electric motor 15. The cathodic high-voltage carbonized fiber 16, fixed at the centre of the front surface of the air outlet grille 2, performs an air ionizing function. On the left and right end of inner housing 25 there are separate catches 17. By pressing them down, users are able to lift up the protective cover 9 in order to replace the air filter 5.

There is a function selector switch 18, a fan speed selector switch 19 and a disinfector selector switch 20 located within the control panel cover 7 on the top of the housing 1. There is a power source input port 10 and rear cover circular hole 11 located within the bottom of the housing 6, which contain rear cover light-emitting diodes 12 of different colors. There are a number of front cover circular holes 13 located on the front surface of the control panel cover 7, which contain front cover light-emitting diodes 21 of different colors, which act as function indicators. There are 3 elongated holes 22, which contain the function indicator light 23, the function selector switch 18, fan speed selector switch 19 and a disinfector selector switch 20. The function selector switch 18 controls the functioning of the electric motor 15, the fan 14, the UVC light source 28 and the carbonized fiber 16. The fan speed selector switch 19 controls the speed of the electric motor 15, the fan 14, and the disinfector selector switch 20 controls the UVC light source 28 and the carbonized fiber 16.

There are neck holes 29 on both side of the rear housing 6 and the front housing 1 and the retractable stand 30 located within the back of the rear housing 6.

The neck holes 29 allow users with the use of neck strap 24 and to pick up the photo-electronic air disinfector easily. The retractable stand 30 contains swivel function which allows the photo-electronic air disinfector to stand on any flat surface.

There is an air guide unit 26 connected to the air inlet grille 4 on the top of the front of the inner housing 25. The air guide unit 26 includes a UVC light source 28, multiple reflecting mirrors 27, the photocatalyst module 31 and the carbonized fiber 16. The multiple reflecting mirrors 27 and the photocatalyst module 31 are opposite to the light source of a UVC light source 28. The multiple reflecting mirrors are to strengthen the illumination range of the opposite light source from a UVC light source 28 and produce reflected UVC rays for two purposes: first repeated irradiation of bacteria and germs and second increased intensity of UV light for the photocatalyst.

On the back of inner housing 3 there is the main electrical function circuit equipment 32 which contain a power source input port 10 and a rear cover light-emitting diodes 12 of different colors.

When the photo-electronic air disinfector is switched on, air containing bacteria, germs and mould is sucked by the fan 14 into air guide unit 26 through the protective cover 9, and the air filter 5. Air streams through the air guide unit 26 before reaching the air outlet grille 2. The UVC light source 28, multiple reflecting mirrors 27, the photocatalyst module 31 are mounted in the centre of the air guide unit 26, where air is disinfected by photocatalysis and the UVC light action before reaching the air outlet grille 2 where negative ions are released to the air by the cathodic high-voltage carbonized fiber 16 prior to eventual discharge from the air outlet grille 2.

The principles of the electrical circuits of the photo-electronic air disinfector are: the external power supply input reaches the main electrical function circuit equipment 32 to recharge the rechargeable battery 33 and via the function selector switch 18, providing a negative high-voltage, and also to supply power to a speed control circuit, which supplies the fan 14, and then to supply power to reach the ionizer circuit and supplies power to the automatic cycle control circuit of the UVC light source 28 activation circuit, controlling the alternating operation of the ionizer and the UVC light source 28. Without the external power supply input reaching the main electrical function circuit equipment 32, the rechargeable battery 33 will provide the power supply instead.

Photocatalysis is the process when photocatalyst like titanium dioxide coming into contact with light including UV light, an oxidation-reduction reaction occurs such that volatile organic compounds (VOC) and various germs (like mould, E.coli, Staphylococcus aureus and others) are broken down and made harmless. Photocatalysis is an effective process for use in air and water disinfection, though feasibility of its use has been limited because coating of a photocatalyst on the wall or furniture is necessary for it to function for human benefit.
To put the photocatalyst to work without coating on the wall or furniture will make the use of photocatalysis more commonplace for air disinfection. Inside the air guide unit of the device, a structure called the photocatalyst module comprising a structure with entire surface area coated with a photocatalyst is positioned by the side of a UVC light source. After absorbing UVC light emitted by the UVC light source, the photocatalyst generates superoxide (O2-) and hydroxyl radical(· OH) with strong oxidation power, which can effectively decompose organic matter and various germs and render them harmless. The process of photocatalysis will therefore take away indoor odour like formaldehyde and deactivate bacteria and viruses by turning them into harmless small molecule water and carbon dioxide.

The efficacy of photocatalysis also depends on and is directly proportional to the intensity of UVC light shining on the photocatalyst. In this device, multiple mirrors are installed on the internal wall of the air guide unit such that UVC emitted from the UVC light source will be reflected repeatedly inside air guide unit and reaching the photocatalyst numerous times and thus increasing the intensity of UVC light shining on the photocatalyst, thereby increasing the efficacy of the photocatalysis for decomposing organic matter and various germs.

The efficacy of the germicidal function of UVC is proven and well known, but the use of UVC for air sterilization is still very limited because UVC can only be safely used in a closed environment to avoid harm to the human body. The invention overcomes the shortcoming that the UVC air purification may cause harm to the human body, through the use of an UVC light source inside an enclosed air guide unit such that the UVC emitted from the source cannot get out of the device to cause any harm to the human body. To increase the efficacy of the sterilization power of UVC, multiple mirrors are installed on the internal wall of the air guide unit and directly facing the UVC light source such that UVC emitted will be reflected again and again by the mirrors inside the air guide unit, so that the bacteria or viruses in air is repeatedly irradiated by UVC through multiple reflections of the UVC by the mirrors.

Negative ions generated by the negative ion generator with a high-voltage carbonized fiber when coming into contact with fine particles in the air will condense them into larger particles which will settle on the ground, thus reducing harmful pollutants in the air. It has been said that an increased level of negative ions in the air caused by the negative ion generator helps boost the biochemical reactions in our body and reduces hormonal secretions held responsible for depression and tiredness.

Usual air purifier will preferably have to be placed in the middle of a room for the benefit of providing fresh air to people inside the room. This photo-electronic air disinfector however is operable by a rechargeable battery, and could be carried around by the user through hanging on the neck of a person by a strap hooked to the device, enabling the user to breathe in purified air coming out of the photo-electronic air disinfector within a short distance. It can be conveniently used in homes, hospitals, offices, shops and transportation for improving air quality. It also helps to revitalize air quality amidst the constant degradation of our natural environment.

## Claims

1. The photo-electronic air disinfector comprises a housing containing a fan, an air guide unit connecting the air inlet and the air outlet, a UVC light source, a photocatalyst module comprising a structure with entire surface area coated with a photocatalyst positioned by the side of the UVC light source, reflecting mirrors installed on the internal walls of the unit facing the UVC light source, producing sterilization effect by photocatalysis:
- wherein the photocatalyst module comprising a structure with entire surface area coated with a photocatalyst positioned by the side of the UVC light source enables photocatalysis triggered by the UVC light to produce oxidation power for decomposing organic matter and various germs and rendering them harmless;
- while multiple mirrors installed on the internal wall of the air guide unit will cause the UVC emitted from the UVC light source to be reflected repeatedly inside the air guide unit and reaching the photocatalyst numerous times and thus increasing the intensity of UVC light shining on the photocatalyst, thereby increasing the efficacy of the photocatalysis for decomposing organic matter and various germs.

2. The disinfector according to claim 1, wherein the multiple reflecting mirrors are installed on the internal wall of the air guide unit and positioned surrounding the UVC light source such that UVC light rays emitted will be reflected back and forth multiple times inside the unit by the reflecting mirrors, such that the bacteria or germs in air are repeatedly irradiated by UVC light through multiple reflections of the UVC light rays by the mirrors.

3. The disinfector according to claim 1, wherein a negative ion generator is installed for producing negative ions to be extracted outside the disinfector by the air flow produced by the fan, such that the negative ions when coming into contact with fine particles in the air will pull the particles together through attraction and condense into larger particles for settling on the ground, thus reducing harmful pollutants in the air;

4. The disinfector according to claim 1, wherein a combination of all the features of the device provides a four-folded mechanisms for disinfection and purification of air, namely: - first, the air filter for filtering the air for dusts, particles, germs before entering the device,
- second, sterilization of the air by photocatalysis produced by the photocatalyst module exposed to direct and reflected UVC light resulting in oxidation process for decomposing organic matter and various germs and rendering them harmless,
- third, sterilization by way of UVC light rays and their multiple reflections through multiple mirrors installed on the internal wall of the air guide unit, enabling multiple bacteria or germs to be repeatedly irradiated by UVC light rays and their multiple reflections, and
- fourth, negative ions generated by the negative ion generator pulling fine particles in the air together through attraction and condense them into larger particles for settling on the ground, thus reducing harmful pollutants in the air.

5. The disinfector according to claim 1, wherein the disinfector is operable by a rechargeable battery and could be carried around by the user through hanging on the neck of a person by a strap hooked to the device, enabling the user to breathe in purified air coming out of the disinfector within a short distance.

6. The disinfector according to claim 1, wherein the housing has retractable stand contain swivel function which allow the disinfector to stand on any flat surface.

7. The disinfector according to claim 1, wherein the photocatalysis function, the UVC germicidal function and the negative ions generating function are all controlled by the same electrical circuit which avail users the choices of operating these functions separately, simultaneously or alternatively.
